**Europäisches Patentamt**

**European Patent Office**

**Office européen des brevets**

(11) Publication number: **0 163 220**
**B1**

(12) **EUROPEAN PATENT SPECIFICATION**

(45) Date of publication of patent specification: **20.06.90**

(21) Application number: **85106127.5**

(22) Date of filing: **18.05.85**

(60) Divisional application 89109006.0 filed on 18/05/85.

(51) Int. Cl.⁵: **G 01 N 33/53,**
**G 01 N 33/543, C 12 Q 1/68**

(54) **Nucleic acid hybridization assay employing detectable anti-hybrid antibodies.**

(30) Priority: **01.06.84 US 616132**
**01.03.85 US 707420**

(43) Date of publication of application:
**04.12.85 Bulletin 85/49**

(45) Publication of the grant of the patent:
**20.06.90 Bulletin 90/25**

(84) Designated Contracting States:
**AT BE CH DE FR GB IT LI LU NL SE**

(56) References cited:
**EP-A-0 133 671**
**EP-A-0 139 489**

**CHEMICAL ABSTRACTS, vol. 70, no. 25, 23th June 1969, page 168, no. 113344d, Columnbus, Ohio, US; E.F. SCHWARTZ et al.: "Antibodies to polydenylate-polyuridylate copolymers as reagents for double-strand RNA and DNA-RNA hybrid complexes", & BIOCHEM. BIOPHYS. RES. COMMUN. 1969, 35(1), 115-20**

(73) Proprietor: **MILES INC.**
**1127 Myrtle Street**
**Elkhart Indiana 46514 (US)**

(72) Inventor: **Carrico, Robert J.**
**54256 Silver Street**
**Elkhart, IN 46514 (US)**

(74) Representative: **Dänner, Klaus, Dr. et al**
**c/o Bayer AG Konzernverwaltung RP Patentabteilung**
**D-5090 Leverkusen 1 Bayerwerk (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 163 220 B1

(56) References cited:

CHEMICAL ABSTRACTS, vol. 98, no. 1, 3rd January 1983, page 112, no. 1157s, Columbus, Ohio, US; A.C. PROOIJEN-KNEGT et al.: "In situ hybridization of DNA sequences in human metaphase chromosomes visualized by an indirect fluorescent immunocytochemical procedure", & EXP. CELL RES. 1982, 141(2), 397-407

BIOCHEMISTRY, vol. 17, no. 26, 26th December 1978, pages 5791-98, American Chemical Society, US; W.E. STUMPH et al.: "Gene enrichment using antibodies to DNA/RNA hybrids: Purification and mapping of dictyostelium discoideum rDNA"

## Description

This invention relates to nucleic acid hybridization assay methods and reagent systems for detecting specific polynucleotide sequences. The principle of nucleic acid hybridization assays was developed by workers in the recombinant DNA field as a means for determining and isolating particular polynucleotide base sequences of interest. It was found that single stranded nucleic acids, e.g., DNA and RNA, such as obtained by denaturing their double stranded forms, will hybridize or recombine under appropriate conditions with complementary single stranded nucleic acids. By labeling such complementary probe nucleic acids with some readily detectable chemical group, it was then made possible to detect the presence of any polynucleotide sequence of interest in a test medium containing sample nucleic acids in single stranded form.

In addition to the recombinant DNA field, the analytical hybridization technique can be applied to the detection of polynucleotides of importance in the fields of human and veterinary medicine, agriculture, and food science, among others. In particular, the technique can be used to detect and identify etiological agents such as bacteria and viruses, to screen bacteria for antibiotic resistance, to aid in the diagnosis of genetic disorders such as sickle cell anemia and thalassemia, and to detect cancerous cells. A general review of the technique and its present and future significance is provided in Biotechnology (August 1983), pp. 471—478.

Background information

The following information is provided for the purpose of making known information believed by the applicant to be of possible relevance to the present invention. No admission is necessarily intended, nor should be construed, that any of the following information constitutes prior art against the persent invention.

The state-of-the-art nucleic acid hybridization assay techniques generally involve immobilization of the sample nucleic acid on a solid support. Hybridization between particular base sequences or genes of interest in the sample nucleic acid is determined by separating the solid support from the remainder of the reaction mixture which contains unbound labeled probe, followed by detection of the label on the solid support.

The need to immobilize sample nucleic acids in order to conduct the state-of-the-art hybridization assay poses two significant problems. Firstly, the procedures required to accomplish immobilization are generally time consuming and add a step which is undesirable for routine use of the technique in a clinical laboratory. Secondly, proteins and other materials in the heterogeneous sample, particularly in the case of clinical samples, can interfere with the immobilization of the nucleic acids.

As alternatives to immobilizing sample nucleic acids and adding labeled probe, one can use an immobilized probe and label the sample nucleic acids in situ, or one can use a dual hybridization technique requiring two probes, one of which is immobilized and the other labeled [Methods in Enzymology 65:468 (1968) and Gene 21:77-86(1983)]. The former alternative, however, is even less desirable since the in situ labeling of the sample nucleic acids requires a high degree of technical skill which is not routinely found in clinical technicians and there are no simple, reliable methods for monitoring the labeling yield, which can be a significant problem if the labeling media contain variable amounts of inhibitors of the labeling reaction. The dual hybridization technique has the disadvantages of requiring an additional reagent and incubation step and the kinetics of the hybridization reaction can be slow and inefficient. The accuracy of the assay can also be variable if the complementary of the two probes with the sample sequence is variable.

Techniques for directly detecting the polynucleotide duplex formed as the product of hybridization between the sample and probe polynucleotides, and thereby dispensing with the chemical labeling and immobilization of sample or probe polynucleotides, have been generally unsatisfactory. Attempts to generate antibodies which will selectively bind double stranded DNA · DNA hybrids over single stranded DNA have failed [Parker and Halloran, "Nucleic Acids in Immunology", ed. Plescia and Braun, Springer-Verlag, NY (1969) pp. 18 et seq]. Some success has been achieved in generating antibodies that will bind DNA · RNA mixed hybrids or RNA · RNA hybrids and have low affinity for the single stranded polynucleotides [see, for example, Rudkin and Stollar, Nature 265:472(1977)]. Rudkin and Stollar fixed whole cells on microscope slides and exposed the DNA in the nucleus. It was hybridized with an RNA probe and the hybrid was detected by fluorescence microscopy with fluorescein-labeled antibody to DNA · RNA. However, these methods are described, as in the case of the hybridization techniques discussed above employing labeled probes, as requiring immobilization of the sample nucleic acids. Immobilization of cellular DNA for in situ hybridization is particularly tenuous because the DNA must remain fixed to delicate cell residues during the hybridization and immunochemical detection steps. The results observed by fluorescence microscopy do not give quantitative data on the amount of hybrid formed.

Specific antibodies to double-strand RNA and DNA-RNA hybrids are known from E. F. Schwartz et al: "Antibodies to polydenylate-polyuridylate copolymers as reagents for double-strand RNA and DNA-RNA hybrid complexes", in Biochem. Biophys. Res. Commun. 1969, 35(1), 115-20.

These antibodies have been used to localize DNA-RNA hybrids on chromosomes (see Prooijen-Knegt et al.' "In situ hybridization of DNA sequences in human metaphase chromosomes visualized by an indirect fluorescent immunocytochemical procedure", in Exp. Cell. Res. 1982, 141(2), 397—407 and Biochemistry, vol 17. no 26,

3 4

26th December 1978, pages 5791—98. American Chamcal Society, U.S.; W.E. Stumpf et al.; "Gene enrichment using antibodies to DNA-RNA hybrids: Purification and mapping of dictyostelium discoideum rDNA").

The antibodies have not been used in hybridization assays.

Accordingly, there is an established need for a nucleic acid hybridization assay which does not require the immobilization or labeling of sample nucleic acids, and which does not require dual probes. Further, such technique should allow the use of a variety of labels, particularly of the nonradioisotopic type. A nucleic acid hybridization assay method and reagent system having these and other advantages are principal objectives of the present invention.

Summary of the invention

A nucleic acid hybridization assay method has now been devised which eliminates the need to immobilize or label sample nucleic acids and which requires but a single probe element. The present invention provides a method for determining a particular polynucleotide sequence in an appropriate test medium containing single stranded nucleic acids. The test medium is combined with an immobilized RNA probe, comprising at least one single stranded base sequence which is substantially complementary to the sequence to be determined, under conditions favorable to hybridization between the sequence to be determined and the complementary probe sequence.

The resulting hybrids can then be detected, after or simultaneously with immobilization of the probe where such was combined with the test medium in an immobilization form, by addition of an antibody reagent capable of binding to the DNA · RNA or RNA · RNA duplexes formed and determining the antibody reagent that becomes bound to such duplexes. A variety of protocols and reagent combinations can be employed in order to carry out the principles of the present method. Important features of the present invention are that the sample nucleic acids are not immobilized or required to be labeled before contact with the probe.

The antibody reagent is the key to specific and sensitive detection of hybridization between the probe and sample nucleic acids. Of course, whole antibodies or appropriate fragments and polyfuncitonal forms thereof can be used as described more fully below, and it will be understood that, when used in this disclosure and the claims which follow, the term antibody reagent will mean whole antibodies and their polyfunctional and fragmented forms as well, unless otherwise noted.

Determination of binding of the antibody reagent to hybridization duplexes can be accomplished in any convenient manner. It is preferred that the antibody, reagent be labeled with a detectable chemical group such as an enzymatically active group, a fluorescer, a chromophore, a luminescer, a specifically bindable ligand, or a radioisotope, the nonradioisotopic labels being especially preferred. The labeled antibody reagent which becomes bound to resulting immobilized hybrid duplexes can be readily separated from that which does not become so bound and the detectable chemical group or label is measured in either separated fraction, usually the former.

By eliminating the need to immobilize or label the sample nucleic acids, the present invention provides a highly advantageous hybridization assay technique. The analyst is not required to have the high level of skill or to take the requisite time to perform the immobilization or labeling procedures. Moreover, there is complete elimination of the potential for sample interferences with the immobilization procedure. The test kit provided to the clinical user would include the probe already immobilized or in a readily immobilizable form such as by binding to an immobilized binding partner. In the prior art systems, interferences from extraneous proteins and other materials in the sample can be a serious problem whether the sample nucleic acids to be immobilized are RNA or DNA.

In the prior art methods, immobilization is accomplished by adsorption onto a microporous membrane, such as nitrocellulose, or by covalent bonding to reactive sites on a solid support. In the first case, proteins from the sample can coat the surface and block the adsorpotion of nucleic acids. Furthermore, many procedures require baking at elevated temperatures, commonly higher than 80°C, in vacuo to fix adsorbed nucleic acids to the support. If mucus or other materials endogeneous to the sample are present, they can become dried to the support to form a film that can adsorb the labeled probe during hybridization and increase the background signal and consequently decrease sensitivity. Also, if an enzyme or other protein is involved in the detection of the label, it can often bind nonspecifically to the film and contribute even further to the background problem. If covalent immobilization is employed, proteins and other materials from the sample can be expected to have available reactive groups which will engage in the coupling reaction and neutralize the coupling of the desired nucleic acids.

Since the present invention provides the probe in preferred embodiments in an already immobilized form or in a form which is reaidly immobilized by binding to an immobilized binding partner, the inefficiencies inherent in the prior art immobilization procedures are overcome and thus the detection limits of the assay are maintained. A further advantage is that nonspecific binding of sample RNA or DNA to the solid support will not be recognized by the antibody reagent. Therefore, the background signal will be low and the detection limit accordingly improved. Relative to the dual hybridization method which uses both a labeled probe and an immobilized probe, the labeled nucleotide can bind nonspecifically to the solid support and contribute background signal. This is not a possibility in the present method since no labeled probe is involved.

Brief description of the drawings

The drawings are schematic representations of preferred methods for performing the present invention. The use of nucleic acid hybridization as an analytical tool is based fundamentally on the double stranded, duplex structure of DNA. The hydrogen bonds between the purine and pyrimidine bases of the respective strands in double stranded DNA can be reversibly broken. The two complementary single strands of DNA resulting from this melting or denaturation of DNA will associate (also referred to as reannealing or hybridization) to reform the duplexed structure. As is now well known in the art, contact of a first single stranded nucleic acid, either DNA or RNA, which comprises a base sequence sufficiently complementary to a second single stranded nucleic acid under apporopriate solution conditions, will result in the formation of, DNA · RNA, or RNA · RNA hybrids, as the case may be.

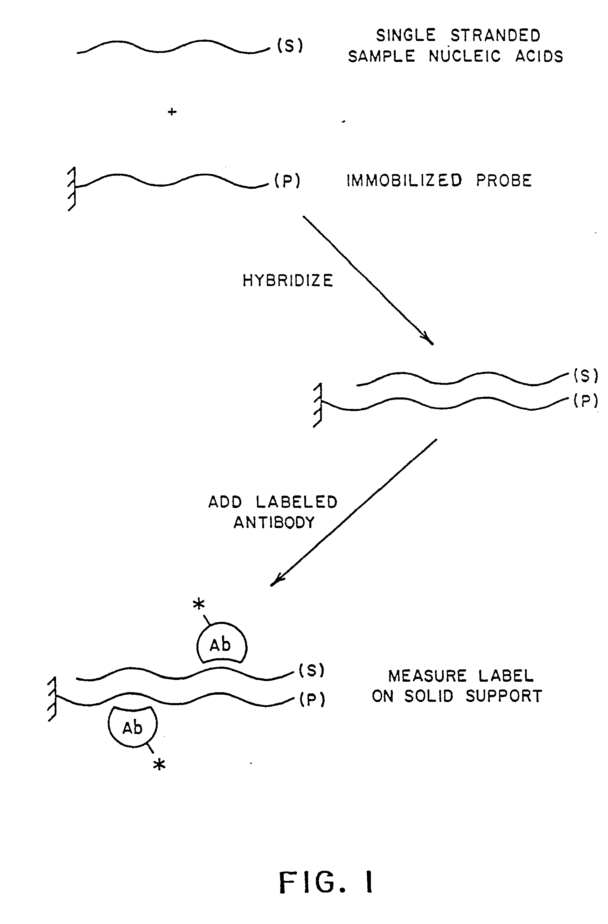

In the embodiment depicted in Fig. 1 of the drawings, the single stranded sample nucleic acids are brought into contact with the immobilized probe under favorable hybridization conditions. The resulting immobilized, hybridized duplexes, optionally after separating such duplexes from the remainder of the reaction mixture, are contacted with a labeled form of antibodies specific for the DNA · RNA or RNA · RNA duplexes. After washing to remove unbound labeled antibody, the label present on the solid support is measured.

Description of the preferred embodiments
The probe

The immobilized RNA probe will comprise at least one single stranded base sequence substantially complementary to the sequence to be detected. However, such base sequence need not be a single continuous polynucleotide segment, but can be comprised of two or more individual segments interrupted by noncomplementary sequences. These nonhybridizable sequences can be linear, or they can be self-complementary and form hairpin loops. In addition, the complementary region of the probe can be flanked at the 3'- and 5'-termini by nonhybridizable sequences, such as those comprising the DNA or RNA of a vector into which the complementary sequence had been inserted for propagation. In either instance, the probe as presented as an analytical reagent will exhibit detectable hybridization at one or more points with sample nucleic acids of interest. Linear or circular single stranded polynucleotides can be used as the probe element, with major or minor portions being duplexed with a complementary polynucleotide strand or strands, provided that the critical homologous segment or segments are in single stranded form and available for hybridization with saample DNA or RNA, and provided that the antibody reagent selected for use with the probe does not significantly crossreact with the double stranded regions in the probie (e.g., where the antibody reagent is specific for DNA · RNA hybrids and the probe comprises RNA · RNA double stranded regions, or vice versa). The complementary probe sequence can be of any convenient or desired length, ranging from as few as a dozen to as many as 10,000 bases, and including oligonucleotides having less than about 50 bases.

The RNA probe can be obtained in a variety of conventional manners. For example RNA can be isolated as the natural products of cells, such as 5s, 16s and 23s ribosomal RNAs from bacteria or cellular transfer RNAs. It is also practical to isolate specific messenger RNAs from cells which specialize in production of large amounts of a protein for which the messenger codes.

In vitro synthesis of RNA probes can be accomplished with a vector which contains the very active Salmonella typhimurium bacteriophage SP6 transcription promoter [Green et al (1983) Cell 32:681]. A vector with multiple restriction endonuclease sites adjacent to the promoter is available from Promega Biotec, Madison, WI. A DNA probe is cloned into the vector which is then propagated in a bacterial host. Multiple RNA copies of the cloned DNA probe can be synthesized in vitro using DNA dependent RNA polymerase from bacteriophage SP6.

It should be understood that in using the expressions "RNA probe" herein, it is not implied that all nucleotides comprised in the probe be ribonucleotides. The fundamental feature of an RNA probe for purposes of the present invention is that it be of such character to enable the stimulation of antibodies to DNA · RNA or RNA · RNA hybrids comprising an RNA probe which do not crossreact to an analytically significant degree with the individual single strands forming such hybrids. Therefore, one or more of the 2'-positions on the nucleotides comprised in the probe can be chemically modified provided the antibody binding characteristics necessary for performance of the present assay are maintained to a substantial degree. Likewise, in addition or alternatively to such limited 2'-deoxy modification, a probe can have in general any other modification along its ribose phosphate backbone provided there is no substantial interference with the specificity of the antibody to the double stranded hybridization product compared to its individual single strands.

Where such modifications exist in an RNA probe, the immunogen used to raise the antibody reagent would preferably comprise one strand having substantially corresponding modifications and the other strand being substantially unmodified RNA or DNA, depending on whether sample RNA or DNA was intended to be detected. Preferably, the modified strand in the immunogen would be identical to the modified strand in an RNA probe. An example of an immunogen is the

hybrid poly(2'-0-methyladenylic acid) · poly(2'-deoxythymidylic acid). Another would be poly(2'-0-ethylinosinic acid) · poly(ribocytidylic acid). The following are further examples of modified nucleotides which could be comprised in a modified probe: 2'-0-methylribonucleotide, 2'-0-ethyl-ribonucleotide, 2'-azidodeoxyribonucleotide, 2'-chlorodeoxyribonucleotide, 2'-0-acetylribonucleotide, and the phosphorothiolates or methylphosphonates of ribonucleotides or deoxyribonucleotides. Modified nucleotides can appear in probes as a result of introduction during enzymic synthesis of the probe from a template. For example, adenosine 5'-0-(1-thiotriphosphate) (ATPαS) and dATPαS are substrates for DNA dependent RNA polymerases and DNA polymerases, respectively. Alternatively, the chemical modification can be introduced after the probe has been prepared. For example, an RNA probe can be 2'-0-acetylated with acetic anhydride under mild conditions in an aqueous solvent [Steward, D. L. et al. (1972) Biochem. Biophys. Acta 262:227].

The critical property of an RNA probe for use herein is that antibodies raised against the probe duplexed with a complementary RNA or DNA strand, as desired, will discriminate in their binding properties between the duplexed form of the probe and single stranded nucleic acids. It is this property which enables detection of hybridized probe in the assay mixture without significant background binding to the unhybridized single stranded form of the probe or any nonspecifically bound single stranded sample nucleic acids. While as described above certain modifications along the ribonucleotide of deoxyribonucleotide strand can be tolerated without loss of antibody discrimination of the duplex from single strands, it will generally be preferable to employ DNA probes which are composed entirely of ribonucleotides when the sample polynucleotide is RNA or DNA.

Immobilization of the probe

As described previously, the probe will be presented for hybridization with sample nucleic acids an immobilized form.

The means by which the probe is ultimately immobilized is not critical to the present invention and any available approach can be taken so long as hybrids formed between the probe and the sequence of interest are rendered immobilized through a property of the probe. Thus, sample nucleic acids are not subjected to direct immobilization.

When presented to the hybridization reaction in an immobilized form, the probe can be in any appropriate form that enables the probe, and any components of the reaction mixture that have become associated therewith by hybridization and/or by binding of the anti-hybrid reagent, to be subsequently isolated or separated from the remaining mixture such as by centrifugation, filtration, chromatography, or decanting. A variety of compositions and configurations of an immobilized probe will thus be evident and available to the worker in the field. Essentially any form of the probe that is insoluble in the reaction mixture can be used. For example, the probe can be aggregated or otherwise precipitated, attached to an insoluble material, polymer, or support, or entrapped in a gel such as agarose or polyacrylamide [see Meth. Enzymol. 12B:635(1968) and PNAS 67:807(1970)]. It is particularly preferred to employ a solid support to which the probe is attached or fixed by covalent or noncovalent bonds, the latter including adsorption methods that provide for a suitably stable and strong attachment. The solid support can take on a variety of shapes and compositions, including microparticles, beads, porous and impermeable strips and membranes, the interior surface of reaction vessels such as test tubes and microtiter plates, and the like. Means for attaching a desired reaction partner to a selected solid support will be a matter of routine skill to the worker in the field.

One method for adsorbing the probe onto nitrocellulose membranes involves saturating a solution of probe with sodium iodide and spotting or filtering aliquots onto the membrane [Bresser et al (1983) DNA 2:243]. The sodium iodide facilitates denaturation of the probe and enhances adsorption onto the membrane. Alternatively, the probe can be treated with glyoxal, usually at concentrations around 1 molar (M), and then adosbred onto the membrane. The probe is fixed by baking at around 80°C under vacuum for a period in the range of 2—4 hours, [Thomas, P.S., (1983) Meth. in Enzymol, 100:255].

Covalent immobilization of RNA probes can also be accomplished. A wide variety of support materials and coupling techniques can be employed. For example, the probe can be coupled to phosphocellulose through phosphate groups activated by carbodiimide or carbonyldiimidazole [Bautz, E.K.F., and Hall, B.D., (1962) Proc Nat'l. Acad. Sci. USA 48:400—408; Shih, T.Y., and Martin, M.A., (1974) Biochem. 13:3411—3418]. Also, diazo groups on m-diazobenzoyloxymethyl cellulose can react with guanine and thymidine residues of the polynucleotide [Noyes, B.E., and Stark, G.R., (1975) Cell 5:301—310; Reiser, J., et al, (1978) Biochem. Biophys. Res. Commun. 85:1104—1112]. Polysaccharide supports can also be used with coupling thorugh phosphodiester links formed between the terminal phosphate of the polynucleotide and the support hydroxyls by water soluble carbodiimide activation [Richwood, D., (1972) Biochim. Biophys. Acta 269:47—50; Gilham, P.T., (1968) Biochem. 7:2809—2813], or by coupling nucleophilic sites on the polynucleotide with a cyanogen bromide activated support [Arndt-Jovin, D.J., et al. (1975) Eur. J. Biochem. 54:411—418; Linberg, U., and Eriksson, S., (1971) Eur. J. Biochem. 18:474—478]. Further, the 3'-hydroxyl terminus of the probe can be oxidized by periodate and ocupled by Schiff base formation with supports bearing amine or hydrazide groups [Gilham, P. T. (1971) Method, Enzymol. 21:191—197; Hansske, H.D., et al, (1979) Method. Enzymol. 59:172—181]. Supports having nucleophilic sites can be reacted with cyanuric chloride and then with the polynucleotide [Hunger, H.D., et al. (1981) Biochim. Biophys. Acta 653:344—349].

In general, any method can be employed for immobilizing the probe, provided that the complementary single stranded sequence is available for hybridization to sample nucleic acids. Particular methods or materials are not critical to the present invention.

A particularly attractive alternative to employing directly immobilized probe is to use an immobilizable form of probe which allows hybridization to proceed to solution where the kinetics are more rapid. Normally in such embodiment, one would use a probe which comprises a reactive site capable of forming a stable covalent or noncovalent bond with a reaction partner and obtain immobilization by exposure to an immobilized form of such reaction partner. Preferably, such reactive site in the probe is a binding site such as a biotin or hapten moiety which is capable of specific noncovalent binding with a binding substance such as avidin or an antibody which serves as the reaction partner.

Essentially any pair of substances can comprise the reactive site/reactive partner pair which exhibit an appropriate affinity for interacting to form a stable bond, that is a linking or coupling between the two which remains substantially intact during the subsequent assay steps, principally the separation and detection steps. The bond formed may be a covalent bond or a noncovalent interaction, the latter being preferred especially when characterized by a degree of selectivity or specificity. In the case of such preferred bond formation, the reactive site on the probe will be referred to as a binding site and the reaction partner as a binding substance with which it forms a noncovalent, commonly specific, bond or linkage.

In such preferred embodiment, the binding site can be present in a single stranded hybridizable portion or in a single or double stranded nonhybridizable portion of the probe or can be present as a result of a chemical modification of the probe. Examples of binding sites existing in the nucleotide sequence are where the probe comprises a promoter sequence (e.g., lac-promoter, trp-promoter) which is bindable by a promoter protein (e.g., bacteriophage promoters, RNA polymerase), or comprises an operator sequence (e.g., lac operator) which is bindable by a repressor protein (e.g., lac repressor), or comprises rare, antigenic nucleotides or sequences (e.g., 5-bromo or 5-iododeoxyuridine, Z-DNA) which are bindable by specific antibodies [see also GB—A—2,125,964]. Binding sites introduced by chemical modification of the polynucleotide comprised in the probe are particularly useful and normally involve linking one member of a specific binding pair to the probe nucleic acid. Useful binding pairs from which to choose include biotin/avidin (including egg white avidin and streptavidin), haptens and antigens/ antibodies, carbohydrates/lectins, enzymes/ inhibitors, and the like. Where the binding pair consists of a proteinaceous member and a nonproteinaceous member, it will normally be preferred to link the nonproteinaceous member to the probe since the proteinaceous member may be unstable under the denaturing conditions of hybridization of the probe. Preferable systems involve linking the probe with biotin or a hapten and employing immobilized avidin or anti-hapten antibody reagent, respectively.

Anti-hybrid antibody reagent and detection schemes

The antibody reagent of the present invention is principally characterized by its ability to bind the DNA · RNA or RNA · RNA hybrids formed between the probe and complementary sample nucleic acids to the significant exclusion of single stranded polynucleotides. As stated previously above, the antibody reagent can consist of whole antibodies, antibody fragments, polyfunctional antibody aggregates, or in general any substance comprising one or more specific binding sites from an antibody for RNA · RNA or DNA · RNA, as the case may be. When in the form of whole antibody, it can belong to any of the classes and subclasses of known immunoglobulins, e.g., IgG, IgM, and so forth. Any fragment of any such antibody which retains specific binding affinity for the hybridized probe can also be employed, for instance, the fragments of IgG conventionally known as Fab, F(ab'), and F(ab')$_2$. In addition, aggregates, polymers, derivatives and conjugates of immunoglobulins or their fragments can be used where appropriate.

The immumoglobulin source for the antibody reagent can be obtained in any available manner such as conventional antiserum and monoclonal techniques. Antiserum can be obtained by well-established techniques involving immunization of an animal, such as a mouse, rabbit, guinea pig or goat, with an appropriate immunogen. The immunoglobulins can also be obtained by somatic cell hybridization techniques, such resulting in what are commonly referred to as monoclonal antibodies, also involving the use of an appropriate immunogen.

Immunogens for stimulating antibodies specific for DNA · RNA hybrids can comprise homopolymeric or heteropolymeric polynucleotide duplexes. Among the possible homopolymer duplexes, particularly preferred is poly-(rA) · poly(dT) [Kitagawa and Stollar (1982) Mol. Immunol. 19:413]. However, in general, heteropolymer duplexes will be preferably used and can be prepared in a variety of ways, including transcription of φD174 virion DNA with RNA polymerase [Nakazato (1980) Biochem, 19:2835]. The selected RNA · DNA duplexes are adsorbed to a methylated protein, or otherwise linked to a conventional immunogenic carrier material, such as bovine serum albumin, and injected into the desired host animal [see also Stollar (1980) Meth. Enzymol. 70:70].

Antibodies to RNA · RNA duplexes can be raised

against double stranded RNAs from viruses such as reovirus or Fiji disease virus which infects sugar cane, among others. Also, homopolymer duplexes such as poly(rI) · poly(rC) or poly-(rA) · poly(rU), among others, can be used for immunization as above.

The binding of the antibody reagent to the hybridized probe duplex according to the present method can be detected by any convenient technique. Advantageously, the antibody reagent will itself be labeled with a detectable chemical group. Such detectable chemical group can be any material having a detectable physical or chemical property. Such materials have been well-developed in the field of immunoassays and in general most any label useful in such methods can be applied to the present invention. Particularly useful are enzymatically active groups, such as enzymes (see Clin. Chem. (1976)22:1243, U.S. Reissue Pat. No. 31,006 and GB—A—2,019,408), enzyme substrates (see US—A—4,492,751, cofactors (see US—A—4,230,797 and 4,238,565), and enzyme inhibitors (see US—A—4,134,792); fluorescers (see Clin. Chem. (1979)25:353); chromophores; luminescers such as chemiluminescers and bioluminescers (see U.S. Pat. No. 4,380,580); specifically bindable ligands such as biotin (see EP—B—63,879) or a hapten (see PCT Publ. 83-2286); and radioisotopes such as $^3$H, $^{35}$S, $^{32}$P, $^{125}$I, and $^{14}$C. Such labels and labeling pairs are detected on the basis of their own physical properties (e.g., fluorescers, chromophores and radioisotopes) or their reactive or binding properties (e.g., enzymes, substrates, cofactors and inhibitors). For example, a cofactor-labeled antibody can be detected by adding the enzyme for which the label is a cofactor and a substrate for the enzyme. A hapten or ligand (e.g., biotin) labeled antibody can be detected by adding an antibody to the hapten or a protein (e.g., avidin) which binds the ligand, tagged with a detectable molecule. Such detectable molecule can be some molecule with a measurable physical property (e.g., fluorescence or absorbance) or a participant in an enzyme reaction (e.g., see above list). For example, one can use an enzyme which acts upon a substrate to generate a product with a measurable physical property. Examples of the latter include, but are not limited to, β-galactosidase, alkaline phosphatase and peroxidase. Other labeling schemes will be evident to one of ordinary skill in the art.

Alternatively, the antibody reagent can be detected based on a native property such as its own antigenicity. A labeled anti-(antibody) antibody will bind to the primary antibody reagent where the label for the second antibody is a conventional label as above. Further, antibody can be detected by complement fixation or the use of labeled protein A, as well as other techniques known in the art for detecting antibodies.

Where the antibody reagent is labeled, as is preferred, the labeling moiety and the antibody reagent are associated or linked to one another by direct chemical linkage such as involving covalent bonds, or by indirect linkage such as by incorporation of the label in a microcapsule or liposome which is in turn linked to the antibody. Labeling techniques are well-known in the art and any convenient method can be used in the present invention.

Reaction mixture

The test sample to be assayed can be any medium of interest, and will usually be a liquid sample of medical veterinary, environmental, nutritional, or industrial significance. Human and animal specimens and body fluids particularly can be assayed by the present method, including urine, blood (serum or plasma), milk, cerebrospinal fluid, sputum, fecal matter, lung aspirates, throat swabs, genital swabs and exudates, rectal swabs, and nasopharnygal aspirates. Where the test sample obtained from the patient or other source to be tested contains principally double stranded nucleic acids, such as contained in cells, the sample will be treated to denature the nucleic acids, and if necessary first to release nucleic acids from cells. Denaturation of nucleic acids is preferably accomplished by heating in boiling water or alkali treatment (e.g., 0.1 N sodium hydroxide), which if desired, can simultaneously be used to lyse cells. Also, release of nucleic acids, can, for example, be obtained by mechanical disruption (freeze/thaw, abrasion, sonication), physical/chemical disruption (detergents such as Triton®, Tween®, sodium dodecylsulfate, alkali treatment, osmotic shock, or heat), or enzymatic lysis (lysozyme, proteinase K, pepsin). The resulting test medium will contain nucleic acids in single stranded form which can then be assayed according to the present hybridization method.

As is known in the art, various hybridization conditions can be employed in the assay. Typically, hybridization will proceed at slightly elevated temperatures, e.g., between about 35 and 75°C and usually around 65°C, in a solution comprising buffer at pH between about 6 and 8 and with appropriate ionic strength (e.g., 2XSSC where 1XSSC=0.15M sodium chloride and 0.015M sodium citrate, pH 7.0), protein such as bovine serum albumin, Ficoll® (a trademark identifying a copolymer of sucrose and epichlorohydrin sold by Pharmacia Fine Chemicals, Piscataway, NY), polyvinylpyrrolidone, and a denatured foreign DNA such as from calf thymus or salmon sperm). The degree of complementarity between the sample and probe strands required for hybridization to occur depends on the stringency of the conditions. The extent and specificity of hybridization is affected by the following principal conditions:

1. The purity of the nucleic acid preparation.

2. Base composition of the probe—G-C base pairs will exhibit greater thermal stability than A-T or A-U base pairs. Thus, hybridizations involving higher G-C content will be stable at higher temperatures.

3. Length of homologous base sequence—Any short sequence of bases (e.g., less than 6 bases), has a high degree of probability of being present in many nucleic acids. Thus, little or no specificity can be attained in hybridizations involving such short sequences. The present homologous probe sequence will be at least 10 bases, usually 20 bases or more, and preferably greater than 100 bases. From a practical standpoint, the homologous probe sequence will often be between 300—1000 nucleotides.

4. Ionic strength—The rate of reannealing increases as the ionic strength of the incubation solution increases. Thermal stability of hybrids also increases.

5. Incubation temperature—Optimal reannealing occurs at a temperature about 25—30°C below the melting temperature (Tm) for a given duplex. Incubation at temperatures significantly below the optimum allows less related base sequences to hybridize.

6. Nucleic acid concentration and incubation time—Normally, to drive the reaction towards hybridization, one of the hybridizable sample nucleic acid or probe nucleic acid will be present in excess, usually 100 fold excess or greater.

7. Denaturing reagents—The presence of hydrogen bond disrupting agents such as formamide and urea increases the stringency of hybridization.

8. Incubation time—The longer the incubation time, the more complete will be the hybridization.

9. Volume exclusion agents—The presence of these agents, as exemplified by dextran and dextran sulfate, are thought to increase the effective concentrations of the hybridizing elements thereby increasing the rate of resulting hybridization.

Normally, the temperature conditions selected for hybridization will be incompatible with the binding of antibody reagent to formed hybrids and detection of the label response. Accordingly, the antibody reagent binding step and label detection step will proceed after completion of the hybridization step. The reaction mixture will usually be brought to a temperature in the range of from about 3°C to about 40°C and the binding and detection steps then performed. Dilution of the hybridization mixture prior to addition of antibody reagent is desirable when the salt and/or formamide concentrations are high enough to interfere significantly with the antibody binding reaction.

It might be found in a particular assay situation using an RNA probe that the probe is subject to partial degradation by alkaline hydrolysis of the phosphodiester bonds or by the presence of ribonucleases. In the former case, hydrolysis can be controlled by avoiding exposure of the probe to a pH higher than about 10. Ribonucleases can be effectively inhibited by the presence of such substances as sodium dodecylsulfate, aurintricarboxylic acid, ribonucleoside vanadyl complexes, heparin, diethylpyrocarbonate, and proteinaceous inhibitors isolated from mammalian sources.

Reagent system

The present invention additionally provides a reagent system, i.e., reagent combination or means, comprising all of the essential elements required to conduct a desired assay method. The reagent system is presented in a commerically packaged form, as a composition or admixture where the compatability of the reagents will allow, in a test device configuration, or more usually as a test kit, i.e., a packaged combination of one or more containers, devices, or the like holding the necessary reagents, and usually including written instructions for the performance of assays. Reagent systems of the present invention include all configurations and compositions for performing the various hybridization formats described herein.

In all cases, the reagent system will comprise (1) an immobilized probe as described herein, and (2) the antibody reagent, preferably labeled with a detectable chemical group. A test kit form of the system can additionally include ancillary chemicals such as the components of the hybridization solution and denaturation agents capable of converting double stranded nucleic acids in a test sample into single stranded form. Preferably, there is included a chemical lysing and denaturing agent, e.g., alkali, for treating the sample to release single stranded nucleic acid therefrom.

The present invention will now be illustrated, but is not intended to be limited, by the following examples.

Example 1
Hybridization assay for detecting bacteriuria using an immobilized RNA probe
A. Preparation of the RNA probe

An 800 base pair fragment of the tuf A gene which encodes for the protein EF-Tu in Escherichia coli is derived from the bacteriophage M13—10 (ATCC 39403-131). The fragment is cloned between Hind III and Eco RI restriction endonuclease sites of M13mp9 (New England Biolabs, Beverly, MA). This plasmid is grown in an E. coli host JM103 (Δlac, pro), supE, thi, strA, sbcB15, hsdR4, F′traD36, proABlac IqZM15. The tuf A fragment is excised from M13-10 and cloned into the Hind III and Eco RI sites of the pSP64 plasmid vector available form Promega Biotec., Madison, WI.

A 15 ml overnight culture of E. coli JM103 carrying the pSP64 plasmid contianing the tuf A fragment is inoculateed into one liter of 2xYT broth in a two liter flask. The culture is incubated at 37°C for 3 hours and the cells are harvested. They are lysed and the DNA is isolated by phenol/chloroform extractions. The closed circular plasmid DNA is purified by centrifugation in a cesium chloride-ethidium bromide gradient [Maniatis, T., Fritsch, E.F. and Sambrook, J., Molecular Cloning, Cold Spring Harbor Laboratory, Cold Spring Harbor, NY (1982)].

The purified plasmid is chromatographed on Sephadex G-50 (Pharmacia Fine Chemicals, Piscataway, NJ) in 10 mM Tris-hydrochloride buffer, pH 7.5, containing 0.1 M NaCl and 1 mM EDTA. The effluent containing DNA is collected and the DNA is preicpitated with cold ethanol. The precipitate is taken up in 10 mM NaCl, 10 mM MgCl₂ and 1 mM dithiothreitol and digested for 1 hour with 1 unit EcoRI per microgram (µg) DNA. Then, the reaction mixture is extracted once with phenol/chloroform and once with chloroform, and the DNA is precipitated with cold ethanol. The precipitate is dissolved in 10 mM Tris-hydrochloride buffer, pH 7.4, to give 500 µg DNA/ml.

A 500 microliter (µl) reaction mixture is prepared with the following composition: 50 µg of the EcoRI digest; 40 mM Tris-hydrochloride buffer, pH 7.5; 6 mM MgCl₂; 2 mM spermine; 0.5 mM ATP, CTP, UTP and GTP; 10 mM dithiothreitol; 500 units RNasin (Promega Biotec) and 50 units of RNA polymerase from bacteriophage SP6 (Promega Biotec). The reaction is allowed to stand for 1 hour at room temperature and then 50 units of additional RNA polymerase is added and allowed to react for another hour.

DNA in the reaction is digested for 10 minutes at 37°C with 10 µg of RNase-fre DNase. The reaction mixture is extracted with phenol/chloroform and chromatographed on Sephadex G-50 in 10 mM Tris-hydrochloride buffer, pH 7.4, 0.1 M NaCl. The RNA is collected and precipitated with cold ethanol. The precipitate is dissolved in 50 mM sodium acetate buffer, pH 5.0, containing 1 mM EDTA.

The RNA probe described above is immobilized on acrylic beads with reactive epoxide groups available under the tradename Eupergit C from Accurate Chemical and Scientific Corp., Westbury, NY. Three milliliters (3 ml) of 50 mM sodium acetate buffer, pH 4.5, containing 250 µg of RNA probe is shaken at room temperature for 10 hours with 200 mg of Eupergit C. The buffer is removed and assayed for RNA to determine the extent of immobilization that has occurred.

The resin is then washed by shaking briefly with 1 ml of 0.1 M sodium phosphate buffer, pH 6.5, containing 1.2 M NaCl, 0.5% (w/v) sodium dodecylsulfate, 1 mg polyvinylpyrrolidone/ml, and 5 mg bovine serum albumin/ml. This hybridization solution is removed and replaced by 1 ml of fresh hybridization solution and the suspension incubated at 65°C for 1 hour to remove noncovalently bound RNA probe. The solution is removed and the resin-RNA probe conjugate is suspended in 50 ml of the hybridization solution.

B. Preparation of methylated thyroglobulin

One hundred milligrams of bovine thyroglobulin (Sigma Chemical Co., St. Louis MO) is combined with 10 ml of anhydrous methanol and 400 µl of 2.55 M HCl in methanol. This mxiture is stirred on a rotary mixer at room temperature for 5 days. The precipitate is collected by centrifugation and washed twice with methanol and twice with ethanol. Then it is dried under vacuum overnight. About 82 mg of dry powder is obtained.

C. Preparation of antibody to DNA · RNA hybrid

A DNA · RNA hybrid is preapred by transcription of φX174 virion DNA and RNA polymerase as described by Nakazato [Biochem. 19:2835(1980)]. One hundred fifty (150) micrograms (mg) of the hybrid in 250 µl of 20 mM Tris-hydrochloride buffer, pH 7.4, 1 mM EDTA is combined with 150 µg of methylated thyroglobulin in 250 µl water. A precipitate forms and is suspended in Tris-buffer. This mxiture is emulsified with an equal volume of Freunds adjuvant. Mice are each immunized with 0.5 ml of the suspension and when serum antibody titers to RNA · DNA develop, hybridomas are prepared and screened for monoclonal antibody specific for RNA · DNA [Stuart et al (1981) Proc. Natl. Acad. Sci. USA 78, 3751, Galfre and Milstein (1981) Meth. in Enzymol. 73, 1].

The cloned hybridomas are propagated in the peritoneal cavity of mice to generate a large quantity of antibody. The ascites fluid is applied to a column of Affigel-Blue resin (Bio-Rad Laboratories, Richmond, VA) equilibrated with 10 mM Tris-hydrochloride buffer, pH 8.0, 0.15 M NaCl. This chromatography removes albumin and the eluted protein which contains the antibody is chromatographed on DEAE-Sepharose (Pharmacia Fine Chemicals). The chromatography is developed with a linear gradient of 10 mM Tris-hydrochloride, pH 8.0, to 10 mM Tris-hydrochloride, pH 8.0, 200 mM NaCl. The major peak of eluted protein contains the monoclonal antibody free of transferrin and albumin.

The most preferred hybridoma is that deposited with the American Type Culture Collection, Rockville, MD as ATCC HB 8730.

D. Preparation of β-galactosidase-antibody conjugate

Sulfhydryl residues on β-galactosidase are exposed by reduction with dithiothreitol. β-galactosidase (30,000 units, grade VIII, Sigma Chemical Co., St. Louis, MO) in 2 ml of 0.1 M N-2-hydroxyethyl-piperazine-N'2-ethane sulfonate (HEPES), pH 7.0, 0.09 M NaCl, is combined with 3.5 µmol of dithiothreitol and allowed to stand at room temperature for 4 hours. The dithiothreitol is removed by chromatography on a 2.5×80 cm column of Sepharose 6B Cl® (Pharmacia Fine Chemicals) in the buffer described above. Fractions containing protein are combined into a pool. The number of moles of sulfhydryl groups per mole of enzyme is measured by the method of Ellman [.Ellman (1959) Arch. Biochem. Biophys. 82, 70].

Succinimidyl-4-(N-maleimidomethyl) cyclohexane-1-carboxylate (SMCC) (Pierce Chemical Co., Rockford, IL), 5.3 mg, is dissolved in 250 µl of anhydrous N,N-dimethylformamide and a 40 µl aliquot is added to 3 ml of 0.1 M HEPES buffer, pH 7.0, 0.15 M NaCl. A 25 µl aliquot of this aqueous solution is added to 825 µl of HEPES/NaCl buffer and 100 µl of 1 mM glutathione. When this reaction mixture has stood at room temperature for 15 minutes, the unreacted glutathione is determined by Ellman's method.

Monoclonal antibody to DNA · RNA is combined with 400 µmol of SMCC in a final volume of 533 µl of HEPES/0.15 M NaCl buffer and allowed to react for 1 hour at 30°C. The reaction mixture is chromatographed on a 1×24 cm column of Biogel P-2 resin (Bio-Rad Laboratories, Richmond, CA) and eluted with HEPES/0.15 M NaCl buffer. Effluent containing protein is pooled, and the protein concentration is determined by the method of Sedmack and Grossberg Anal. Biochem. 79, 544(1977)] and the number of maleimide groups determined by titration with glutathione as described above.

A 2.8 mg portion of the antibody-maleimide adduct is combined with 10 mg of dithiothreitol treated β-galactosidase and allowed to react for 4 hours at room temperature. The reaction mixture is chromatographed at 4°C on a 2.5×80 cm column of Sepharose 6B Cl in HEPES/0.15 M NaCl at 4°C. The flow rate is set at 4 ml/hour and 3 ml fractions are collected. Fractions are assayed for β-galactosidase activity and antibody binding activity. Fractions having both activities are pooled.

E. Hybridization assay

Ten milliliter aliquots of urine from patients with possible uninary tract infections are centrifuged at 10,000×g for ten minutes and the supernatants decanted and discarded. The sediments are suspended in 50 µl of 10 mM Tris-hydrochloride buffer, pH 8.0, containing 20 mg of egg white lysozyme/ml (Sigma Chemical Co., St. Louis, MO), 0.1 M NaCl, and 5 mM EDTA. The reaction is allowed to stand at room temperature for 30 mintues and then 10 µl of 1 M NaOH is added and this alkaline mixture is allowed to stand at room temperature for 10 mintues to denature DNA from any bacteria in the original specimen. The reaction mixtures are neutralized by addition of 250 µl of the buffered suspension of the resin-RNA conjugate described previously. This hybridization system is incubated at about 65°C for 15 hours with gentle agitation.

The resin-RNA probe conjugate is allowed to settle and the liquid is decanted. The resin is washed twice by suspension in 0.5 ml each time of 0.1 M sodium phosphate buffer, pH 7.4, 5 mg bovine serum albumin/ml. The resin is combined with 300 µl of 0.1 M sodium phosphate buffer, pH 7.4, containing 10 mM MgCl$_2$, 5 mg bovine serum albumin/ml, and 0.4 µg β-glaactosidase-antibody/ml (anti-DNA · RNA). The mixture is agitated gently for 1 hour at room temperature and the resin washed twice, one minute each, with 5 ml of 0.1 M sodium phosphate buffer, pH 7.4, containing 0.1% Tween 20 detergent. The washed resin is agitated gently for 30 minutes at room temperature in 1.0 ml of 0.1 M sodium phosphate buffer, pH 7.4, containing 800 µM 7-β-galactosyl-3-[6-aminohexylcarboxamide]coumarin [Worah et al (1981) Clin. Chem. 27:673]. At the end of this incubation, the fluorescence of the solution is recorded using 400 nanometers (nm) excitation and 450 nm emission.

Fluorescence signals developed with urine specimens containing greater than 100,000 bacteria per ml will be significantly higher than those containing less than 5000 bacteria per ml. This method can be used as a qualitative test for bacterirria.

## Claims

1. A method for determining a particular polynucleotide sequence in a test medium containing single stranded nucleic acids, comprising the steps of combining the test medium with a probe comprising at least one single stranded base sequence which is substantially complementary to the sequence to be determined under conditions favorable to hybridization between the sequence to be determined and the complementary probe sequence, and detecting laybridized probe by binding of an antibody reagent capable of binding to DNA/RNA or RNA/RNA duplexes formed between the sequence to be determined and the complementary probe sequence and determining the antibody reagent that becomes bound to such duplexes, characterized in that the probe is an immobilized RNA probe.

2. The method of claim 1 wherein the antibody reagent is labeled with a detectable chemical group selected from an enzymatically active group, a fluorescer, a chromophore, a luminescer, a specifically bindable ligand, or a radioisotope.

3. The method according to any of claims 1 and 2 wherein the test medium comprises a biological sample which has been subjected to conditions to release and denature nucleic acids present therein.

4. A reagent system for detecting a particular polynucleotide sequence in a test medium by nucleic acid hybridization, comprising (i) a polynucleotide probe having at least one single stranded by sequence which is substantially complementary to the sequence to be determined, and (ii) an antibody reagent capable of binding to DNA/RNA or RNA/RNA duplexes formed between the sequence to be determined and the complementary probe sequence, characterized in that the probe is an immobilized RNA probe.

5. The reagent system of claim 4 wherein the probe is immobilized by being fixed to a solid support.

6. The reagent system of claim 4 or 5 wherein the antibody reagent is labaled with a detectable chemical group selected from an enzymatically active group, a fluorescer, a chromophore, a luminescer, a specifically bindable ligand, or a radioisotope.

7. A reagent system according to any of claims 4 to 6 wherein the antibody reagent comprises a monoclonal antibody produced by a hybridoma cell line having the characteristics of cell line HB 8730 deposited with the American Type Culture Collection.

**Patentansprüche**

1. Methode zur Bestimmung einer bestimmten Polynukleotidsequenz in einem Testmedium, das einzelsträngige Nukleinsäuren enthält, welche die folgenden Schritte umfasst:

Vereinigen des Testmediums mit einer Sonde, umfassend wenigstens eine einzelsträngige Basensequenz umfasst, die im wesentlichen zu der zu bestimmenden Sequenz unter für eine Hybridisierung zwischen der zu bestimmenden und der komplementären Sondensequenz geeigneten Bedingungen komplementär ist, und Nachweis der hybridisierten Sonde durch Binden eines Antikörperreagens, das in der Lage ist, an DNA · RNA- oder RNA · RNA- Duplexe, wie sie sich zwischen der zu bestimmenden und der komplementären Sondensequenz ausbilden, zu binden, und

Bestimmung des Antikörperreagens, das an solche Duplexe gebunden wird,

dadurch gekennzeichnet, dass die Sonde eine immobilisierte RNA-Sonde ist.

2. Methode nach Anspruch 1, wobei das Antikörperreagens mit einer nachweisbaren chemischen Gruppe, ausgewählt aus einer enzymatisch aktiven Gruppe, einem Fluoreszierstoff, einem Chromophor, einem Lumineszierstoff, einem spezifisch bindungsfähigen Liganden oder einem Radioisotop, markiert ist.

3. Methode nach einem der Ansprüche 1 und 2, wobei das Testmedium eine biologische Probe umfasst, das Bedingungen zur Freisetzung und Denaturierung der darin anwesenden Nukleinsäuren unterworfen wurde.

4. Reagenssystem zum Nachweis einer bestimmten Polynukleotidsequenz in einem Testmedium durch Nukleinsäurehybridisierung, umfassend (i) eine Polynukleotid-Sonde, die wenigstens eine einzelsträngige Sequenz aufweist, die im wesentlichen komplementär zu der zu bestimmenden Sequenz ist, und (ii) ein Antikörperreagens, das in der Lage ist, an DNA · RNA- oder RNA · RNA-Duplexe, die sich zwischen der zu bestimmenden Sequenz und der komplementären Sondensequenz ausbilden, zu binden, dadurch gekennzeichnet, dass die Sonde eine immobilisierte RNA-Sonde ist.

5. Reagenssystem nach Anspruch 4, in welchem die Sonde durch Fixierung an einen festen Träger immobilisiert ist.

6. Reagenssystem nach Ansprüchen 4 oder 5, in welchem das Antikörperreagens mit einer nachweisbaren chemischen Gruppe, ausgewählt aus einer enzymatisch aktiven Gruppe, einem Fluoreszierstoff, einem Chromophor, einem Lumineszierstoff, einem spezifisch bindungsfähigen Liganden oder einem Radioisotop, markiert ist.

7. Reagenssystem nach einem der Ansprüche 4 bis 6, in welchem das Antikörperreagens einen monoklonalen Antikörper umfasst, der von einer Hybridomzellinie hergestellt ist, die die Charakteristika der bei der American Type Culture Collection hinterlegten Zellinie HB 8730 hat.

**Revendications**

1. Procédé pour la détermination d'une séquence particulière de polynucléotides dans un milieu d'essai contenant des acides nucléiques à un seul brin, ce procédé comprenant les étapes consistant à combiner le milieu d'essai avec une sonde comprenant au moins une séquence de bases à un seul brin qui est pratiquement complémentaire à la séquence devant être déterminée dans des conditions favorables à l'hybridation entre la séquence à déterminer et la séquence complémentaire de sondes, puis détecter la sonde hybridée par fixation d'un réactif anticorps capable de se fixer à des duplex d'ADN/ARN ou d'ARN/ARN formés entre la séquence à déterminer et la séquence complémentaire de sondes, puis déterminer le réactif anticorps venant se lier à ces duplex, caractérisé en ce que la sonde est une sonde immobilisée d'ARN.

2. Procédé selon la revendication 1, dans lequel le réactif anticorps est marqué par un groupe chimique détectable choisi parmi un groupe à activité enzymatique, un agent fluorescent, un chromophore, un agent luminescent, un coordinat spécifiquement fixable ou un radio-isotope.

3. Procédé selon l'une quelconque des revendications 1 et 2, caractérisé en ce que le milieu d'essai comprend un échantillon biologique qui a été soumis à des conditions permettant la libération et la dénaturation des acides nucléiques qui y sont présents.

4. Système de réactifs en vue de détecter une séquence particulière de polynucléotides dans un milieu d'essai par hybridation d'acides nucléiques, ce système comprenant (i) une sonde de polynucléotides comportant au moins une séquence à un seul brin qui est pratiquement complémentaire à la séquence à déterminer et (ii) un réactif anticorps capable de se fixer aux duplex d'ADN/ARN ou d'ARN/ARN formés entre la séquence à déterminer et la séquence de sondes complémentaires, caractérisé en ce que la sonde est une sonde immobilisée d'ARN.

5. Système de réactifs selon la revendication 4, caractérisé en ce que la sonde est immobilisée en étant fixée à un support solide.

6. Système de réactifs selon la revendication 4 ou 5, caractérisé en ce que le réactif anticorps est marqué par un groupe chimique détectable choisi parmi un groupe à activité enzymatique, un agent fluorescent, un chromophore, un agent luminescent, un coordinat pouvant être spécifiquement fixé ou un radio-isotope.

7. Système de réactifs selon l'une quelconque des revendications 4 à 6, caractérisé en ce que le réactif anticorps comprend un anticorps monoclonal produit par une ligne de cellules hybridomes ayant les caractéristiques de la ligne de cellules HB 8730 déposée à l'American Type Culture Collection.

FIG. 1